(19) Europäisches Patentamt
European Patent Office
Office européen des brevets

(11) Numéro de publication: 0 259 233 B1

# (12) FASCICULE DE BREVET EUROPEEN

(45) Date de publication de fascicule du brevet: 25.09.91

(51) Int. Cl.5: C07C 273/18, C07C 275/62

(21) Numéro de dépôt: 87420196.5

(22) Date de dépôt: 10.07.87

## (54) Procédé de préparation de polyisocyanate à groupement biuret.

(30) Priorité: 03.09.86 FR 8612524

(43) Date de publication de la demande: 09.03.88 Bulletin 88/10

(45) Mention de la délivrance du brevet: 25.09.91 Bulletin 91/39

(84) Etats contractants désignés: AT BE CH DE ES FR GB GR IT LI LU NL SE

(56) Documents cités: EP-A- 0 157 088

(73) Titulaire: RHONE-POULENC CHIMIE
25, quai Paul Doumer
F-92408 Courbevoie Cédex(FR)

(72) Inventeur: Robin, Jean
21, rue Duguesclin
F-69006 Lyon(FR)
Inventeur: Carlo, Michel
188, route de Vienne
F-69008 Lyon(FR)
Inventeur: Lavorel, Jacques
Le Bois de Laie
F-01700 Neyron(FR)

(74) Mandataire: Dutruc-Rosset, Marie-Claude et al
RHONE-POULENC CHIMIE Service Brevets
Chimie 25, Quai Paul Doumer
F-92408 Courbevoie Cédex(FR)

EP 0 259 233 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne un procédé de préparation de polyisocyanate à groupement biuret, plus particulièrement à partir d'un diisocyanate aliphatique, cycloaliphatique ou arylaliphatique et d'un agent de biurétisation.

Les polyisocyanates à groupement biuret peuvent être utilisés pour préparer des mousses, des adhésifs et des peintures.

Parmi ces applications, l'utilisation de ces polyisocyanates comme constituants des peintures devient de plus en plus importante, notamment pour l'industrie automobile.

Les pellicules de peintures préparées à partir de polyisocyanates aromatiques jaunissent et se fendillent.

Par contre, les pellicules de peintures préparées à partir de polyisocyanates aliphatiques, cycloaliphatiques ou arylaliphatiques conservent leurs propriétés pendant de très longues durées et sont donc particulièrement adaptées pour les véhicules automobiles.

Cependant, la préparation des polyisocyanates aliphatiques, cycloaliphatiques et arylaliphatiques, au moyen de diisocyanate et d'eau, présente certains inconvénients.

Ainsi, selon les brevets américains No. 3.124.605 et 3.902.127, la réaction du diisocyanate monomère et de l'eau produit de la polyurée qui précipite. La réaction entre le diisocyanate monomère et l'eau qui se dissout en faibles quantités dans ledit diisocyanate, produit le polyisocyanate à groupement biuret. Mais la réaction de l'eau avec les faibles quantités de diisocyanate monomère qu'elle peut dissoudre, conduit à de la polyurée.

Dans le brevet français publié sous le numéro 2.382.468, il a été proposé, comme solution à ce problème, de préparer ces polyisocyanates par réaction du diisocyanate monomère sur l'eau, à une température d'au moins 70 °C et dans un mélange d'un dérivé de l'éthylèneglycol, tel qu'un éther méthylique de l'acétate d'éthylèneglycol, et d'un phosphate de méthyle ou d'éthyle.

Ce procédé permet de supprimer pratiquement la précipitation de polyurée dans le polyisocyanate.

Il est également connu par ailleurs, selon le document EP-A 0 157 088 un procédé de préparation de polyisocyanates à groupement biuret qui consiste à faire réagir un diisocyanate aliphatique avec de l'eau, à température élevée et en présence d'un acide acétique α, α, α tri-substitué ou son anhydride et, plus particulièrement l'acide pivalique.

Cependant il s'avère, lors du stockage de quelques jours des solutions de polyisocyanates à groupement biuret ainsi préparées, que leur viscosité augmente, qu'il se forme des polybiurets et que la teneur en diisocyanate monomère libre augmente.

Pour pallier ces inconvénients et permettre ainsi de préparer et de conserver des polyisocyanates à groupement biuret ayant la viscosité souhaitée, il a maintenant été trouvé un procédé de préparation de polyisocyanates à groupement biuret par réaction d'au moins un diisocyanate monomère aliphatique, cycloaliphatique ou arylaliphatique et d'un agent de biurétisation à une température d'au moins 70 °C, caractérisé en ce que l'on opère en présence d'au moins un acide carboxylique, soluble dans le milieu réactionnel, de formule générale (I) :

$$R_1 - \underset{\underset{\displaystyle R_2}{|}}{CH} - COOH \qquad (I)$$

dans laquelle :

- $R_1$ représente :
  - . un atome d'hydrogène,
  - . un groupement alkyle, linéaire ou ramifié, ayant 1 à 10 atomes de carbone ;
  - . un groupement carboxyle,
  - . un groupement hydroxycarbonylalkyle, dont la partie alkyle, linéaire ou ramifiée, comporte 1 à 9 atomes de carbone,
  - . un groupement alcoxycarbonylalkyle, dont la partie alkyle, linéaire ou ramifiée, comporte 1 à 9 atomes de carbone,
- $R_2$ représente :
  - . un atome d'hydrogène,

. un groupement alkyle ayant 1 à 4 atomes de carbone,

- $R_1$ et $R_2$ peuvent former ensemble et avec l'atome de carbone auquel ils sont liés un cycle cyclaoliphatique ayant 5 ou 6 atomes de carbone, qui peut être substitué par :
  . un ou deux radical alkyles, linéaires ou ramifiés, ayant 1 à 4 atomes de carbone,
  . un ou deux radicaux alcoxy, linéaires ou ramifiés, ayant 1 à 4 atomes de carbone,
  . un groupement carboxyle,
  . un groupement alcoxycarbonyle dont la partie alcoxy comporte 1 à 4 atomes de carbone,
  . un groupement acyle ayant 1 à 4 atomes de carbone,
  . un groupement acétoxy ;

ou de formule (II) :

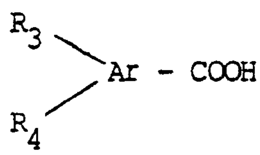

(II)

dans laquelle :

- Ar représente un cycle benzènique, pyridinique ou cyclopentanone,
- $R_3$ et $R_4$, identiques ou différents représentent :
  . un atome d'hydrogène ;
  . un radical alkyle, linéaire ou ramifié, ayant 1 à 4 atomes de carbone,
  . un radical alcoxy, linéaire ou ramifié, ayant 1 à 4 atomes de carbone,
  . un atome d'halogène,
  . un groupement alcoxycarbonyle dont la partie alcoxy comporte 1 à 4 atomes de carbone,
  . un groupement acyle ayant 1 à 4 atomes de carbone,
  . un groupement acétoxy,

et/ou d'au moins un anhydride des acides de formule (I) ou (II).

Parmi les acides carboxyliques de formule (I), on peut citer notamment l'acide acétique, l'acide propionique, l'acide butyrique, l'acide isobutyrique, l'acide hexanoïque, l'acide octanoïque, l'acide dodécanoïque, l'acide malonique, l'acide succinique, l'acide glutarique, l'acide adipique, l'acide subérique, l'acide sébacique, l'acide cyclohexanecarboxylique.

Parmi les acides carboxyliques de formule (II), on peut citer plus particulièrement l'acide benzoïque, l'acide acétylsalicyliqe, l'acide paratertiobutylbenzoïque, l'acide chlorobenzoïque, l'acide diméthoxy-3,4 benzoïque, l'acide chloro-2 benzoïque, l'acide chloro-4 benzoïque, l'acide nicotinique, l'acide isonicotinique, l'acide cyclopentanone-2 carboxylique.

L'acide acétique, l'acide propionique, l'acide butyrique, l'acide isobutyrique, l'acide benzoïque et l'anhydride acétique sont plus particulièrement utilisés pour leur efficacité et leur disponibilité dans le commerce.

La quantité d'acide carboxylique de formule (I) ou (II) et/ou d'anhydride d'un tel acide représente de 0,005 à 0,5 %, de préférence de 0,01 à 0,5 % du poids du diisocyanate monomère.

L'agent de biurétisation utilisé peut être tout composé capable de réagir avec les fonctions isocyanate pour former un biuret. On peut utiliser par exemple le tertiobutanol ou l'eau.

L'eau est l'agent de biurétisation qui est préféré dans le cadre du procédé de l'invention.

Les diisocyanates monomères qui peuvent être utilisés dans le présent procédé sont les diisocyanates aliphatiques, les diisocyanates cycloaliphatiques et les diisocyanates arylaliphatiques dont les groupements isocyanate ne sont pas directement liés à un cycle aromatique.

A titre d'exemples non limitatifs de ces diisocyanates monomères, on peut citer :

- le diisocyanato-1,3 propane,
- le diisocyanato-1,4 butane,
- le diisocyanato-1,5 pentane,
- le diisocyanato-1,6 hexane,
- le diisocyanato-1,4 éthyl-2 butane,
- le diisocyanato-1,5 méthyl-2 pentane,
- le diisocyanato-1,6 triméthyl-2,2,4 hexane,
- le diisocyanato-1,6 triméthyl-2,4,4 hexane,
- le diisocyanato-1,2 cyclohexane

- le diisocyanato-1,4 cyclohexane,
- le bis(isocyanatométhyl)-1,2 cyclobutane,
- le bis(isocyanato-4 cyclohexyl)méthane,
- le triméthyl-3,3,5 isocyanatométhyl-5 isocyanato-1 cyclohexane,
- le bis(isocyanatcméthyl)-1,4 benzène,
- le bis(isocyanatométhyl)-1,2 benzène.

Les diisocyanates monomères peuvent être utilisés séparément ou sous forme de mélanges de plusieurs d'entre eux.

Ainsi par exemple le diisocyanato-1,6 hexane, qui est l'un des diisocyanates monomères préférés, peut être utilisé seul ou en mélanges, notamment avec le diisocyanato-1,5 méthyl-2 pentane et/ou le diisocyanato-1,4 éthyl-2 butane ; des mélanges de ces deux derniers diisocyanates peuvent également être utilisés.

Le rapport molaire diisocyanate monomère/eau peut varier dans de très larges limites. Cependant la masse moléculaire du polyisocyanate obtenu est d'autant plus élevée que le rapport molaire diisocyanate monomère/eau plus bas. Généralement il n'est pas favorable d'avoir une masse moléculaire et une viscosité trop élevées.

D'autre part la masse moléculaire et la viscosité du polyisocyanate obtenu sont d'autant plus faibles que le rapport diisocyanate monomère/eau est plus élevé. Mais un rapport trop élevé n'est pas intéressant économiquement, car cela implique la séparation et la récupération d'une quantité importante de diisocyanate monomère.

C'est pourquoi on adopte généralement un rapport molaire diisocyanate monomère/eau compris entre 2 et 40. De préférence ce rapport est de 5 à 15.

La température, à laquelle est opérée la réaction du diisocyanate monomère et de l'eau, est en général comprise entre 70 °C et 200 °C.

Des températures plus élevées ne gênent pas la réaction, mais elles conduisent à des colorations indésirables.

De préférence on opère entre 110 °C et 150 °C.

Généralement on met en oeuvre le procédé en présence d'un solvant ou d'un mélange de solvants.

La quantité de solvant représente habituellement de 10 % à 80 % en poids du mélange réactionnel.

Comme solvant, on peut notamment utiliser un alcoxyalcane dérivant par exemple de l'éthylèneglycol (ou de l'oxyde d'éthylène) ou du propylèneglycol (ou de l'oxyde de propylène) ; on peut aussi utiliser un carboxylate, en particulier un acétate, d'alcoxyalcane ou de diol.

A titre d'exemples on peut citer les dérivés de l'éthylèneglycol décrits dans le brevet français publié sous le numéro 2.382.468.

On peut utiliser plus particulièrement l'acétate de méthoxy-2 éthyle, le diméthoxy-1,2 éthane, l'acétate d'éthoxy-2 éthyle, le diacétate de l'éthylèneglycol, l'acétate de méthoxy-1 propyle-2, l'acétate de méthoxy-2 propyle-1, l'acétate d'éthoxy-1 propyle-2, l'acétate d'éthoxy-2 propyle-1.

Le solvant utilisé peut être également un ester méthylique et/ou éthylique de l'acide phosphorique, notamment le phosphate de triméthyle, le phosphate de triéthyle, le phosphate de diméthyle et d'éthyle ou le phosphate de diéthyle et de méthyle.

Les solvants utilisés pouvant l'être séparément on en mélanges tels que par exemple des mélanges d'un alcoxylalcane ou d'un carboxylate d'alcoxyalcane ou d'un dicarboxylate de diol avec un ester méthylique et/ou éthylique de l'acide phosphorique.

On peut également, sans sortir du cadre de la présente invention mettre en oeuvre le présent procédé sans solvant ou avec de faibles quantités de solvant.

Par faibles quantités de solvant, on entend des quantités représentant de 1 à 10 fois le poids de l'eau engagée.

De préférence ces quantités représentent de 1 à 5 fois le poids de l'eau engagée.

Ces faibles quantités de solvant ont pour but de diluer l'eau et favoriser son contact et donc sa réaction avec le diisocyanate monomère.

Une variante du présent procédé consiste à opérer une pression absolue supérieure ou égale à 1,2 bar avec une pression partielle d'au moins 0,2 bar de gaz carbonique.

Dans cette variante, on peut en pratique soit purger préalablement l'appareillage à l'aide de gaz carbonique et après avoir mis au moins 1,2 bar de pression initiale absolue, laisser croître au cours de la réaction de biurétisation la pression jusqu'à la valeur désirée, par exemple entre 1,2 et 100 bars absolus ou, après une purge à l'aide d'un gaz inerte sec, compléter la pression dans l'appareillage à l'aide de gaz carbonique jusqu'à une pression initiale absolue d'au moins 1,2 bar et poursuivre comme indiqué précédemment.

Cette variante peut être utilisée avec ou sans solvant, mais son intérêt essentiel réside dans le fait de travailler sans solvant ou avec de faibles quantités de solvant, ce qui augmente largement la productivité de l'appareillage.

En pratique on peut réaliser le procédé selon l'invention en chargeant dans un appareillage adapté, le ou les diisocyanates monomères (le plus souvent le diisocyanato-1,6 hexane), l'acide carboxylique de formule (I) ou (II) ou l'anhydride d'un tel acide et le cas échéant le ou les solvants.

Après chauffage sous agitation entre 70 °C et 200 °C, on ajoute l'agent de biurétisation qui est l'eau le plus souvent.

On peut soit laisser dégager le gaz carbonique formé, soit maintenir une pression de gaz carbonique supérieure à la pression atmosphérique, par exemple 2 à 10 bars.

Après la fin de la réaction de biurétisation (quelques minutes à plusieurs heures généralement), on dégaze si nécessaire le gaz carbonique et on élimine rapidement le solvant éventuel et l'excès de diisocyanate.

On obtient un biuret limpide sans précipité. Sa viscosité est fonction du rapport molaire diisocyanate monomère/agent de biurétisation adopté.

Le biuret est stable dans le temps ; on ne note pas de précipitation après plusieurs mois de stockage, tandis que le taux du diisocyanate libre évolue dans le temps beaucoup moins rapidement que pour les biurets préparés sans acide carboxylique.

Les exemples qui suivent illustrent l'invention.

EXEMPLES 1 et 2 et ESSAI TEMOIN

Dans un ballon tricol de 2 litres muni d'une agitation, d'une régulation de température et d'un réfrigérant à reflux, on charge tous les constituants : diisocyanato-1,6 hexane (HDI) + solvants + eau + acide.

On chauffe jusqu'à 130 °C par l'intermédiaire d'un chauffe ballon.

Le gaz formé à partir de 90 °C se dégage par le réfrigérant. On maintient la température (130 °C) pendant 3 heures.

Le produit final (biuret + HDI + solvant) est évaporé au moyen d'un appareil à film agité.

Le tableau ci-après rassemble les données des essais.

| | Essai témoin sans acide | Exemple 1 avec acide acétique | Exemple 2 avec acide benzoïque |
|---|---|---|---|
| HDI | 904,5 g | 904,5 g | 904,5 g |
| $H_2O$ | 12 g | 12 g | 12 g |
| Solvants (1) | 446 g | 446 g | 446 g |
| Acide | 0 | 0,3 g | 1,0 g |
| Viscosité de biuret à 25°C (en Pascal x s) | 3,8 | 3,8 | 3,4 |
| Stabilité : HDI % (2) | 1,3 | 0,7 | 0,45 |

(1) solvants 1/1 acétate méthoxy-2 éthyle et phosphate d'éthyle

(2) HDI formé après stockage 1 mois à 60°C.

On constate en opérant en présence d'acide (et pour un même rapport HDI/eau) une viscosité plus faible et une amélioration de la stabilité.

## Revendications

**1.** Procédé de préparation d'un polyisocyanate organique à groupement biuret consistant à faire réagir au moins un diisocyanate aliphatique, cycloaliphatique ou arylaliphatique et un agent de biurétisation à une température d'au moins 70° C, caractérisé en ce que l'on opère en présence d'une quantité efficace d'au moins un acide carboxylique de formule générale (I) :

$$R_1 - \underset{\underset{R_2}{|}}{CH} - COOH \qquad (I)$$

dans laquelle :

- $R_1$ représente :
  - . un atome d'hydrogène,
  - . un groupement alkyle, linéaire ou ramifié, ayant 1 à 10 atomes de carbone ;
  - . un groupement carboxyle,
  - . un groupement hydroxycarbonylalkyle, dont la partie alkyle, linéaire ou ramifiée, comporte 1 à 9 atomes de carbone,
  - . un groupement alcoxycarbonylalkyle, dont la partie alkyle, linéaire ou ramifiée, comporte 1 à 9 atomùes de carbone,
- $R_2$ représente :

. un atome d'hydrogène,
. un groupement alkyle ayant 1 à 4 atomes de carbone,

- $R_1$ et $R_2$ peuvent former ensemble et avec l'atome de carbone auquel ils sont liés un cycle cycloaliphatique ayant 5 ou 6 atomes de carbone, qui peut être substitué par :

. un ou deux radical alkyles, linéaires ou ramifiés, ayant 1 à 4 atomes de carbone,
. un ou deux radicaux alcoxy, linéaires ou ramifiés, ayant 1 à 4 atomes de carbone,
. un groupement carboxyle,
. un groupement alcoxycarbonyle dont la partie alcoxy comporte 1 à 4 atomes de carbone,
. un groupement acyle ayant 1 à 4 atomes de carbone,
. un groupement acétoxy ;

ou de formule (II) :

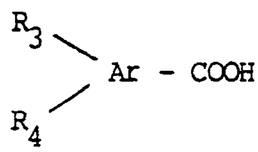

(II)

dans laquelle :

- Ar représente un cycle benzènique, pyridinique ou cyclopentanone,
- $R_3$ et $R_4$, identiques ou différents représentent :

. un atome d'hydrogène ;
. un radical alkyle, linéaire ou ramifié, ayant 1 à 4 atomes de carbone,
. un radical alcoxy, linéaire ou ramifié, ayant 1 à 4 atomes de carbone,
. un atome d'halogène,
. un groupement alcoxycarbonyle dont la partie alcoxy comporte 1 à 4 atomes de carbone,
. un groupement acyle ayant 1 à 4 atomes de carbone,
. un groupement acétoxy,

et/ou d'au moins un anhydride des acides de formule (I) ou (II), et en ce que la quantité d'acide carboxylique de formule (I) ou (II) et/ou l'anhydride d'un tel acide représente de 0,005 à 0,5 %, de préférence de 0,01 à 0,5 %, du poids du diisocyanate monomère.

**2.** Procédé selon la revendication 1, caractérisé en ce que l'agent de biurétisation utilisé est l'eau.

**3.** Procédé selon la revendication 2, caractérisé en ce que le rapport molaire diisocyanate monomère/eau est compris entre 2 et 40 et de préférence entre 5 et 15.

**4.** Procédé selon l'une des revendications 1 à 3, caractérisé en ce que l'acide carboxylique ou l'anhydride est choisi parmi l'acide acétique, l'acide propionique, l'acide isobutyrique, l'acide benzoïque et l'anhydride acétique.

**5.** Procédé selon l'une des revendications 1 à 4, caractérisé en ce que l'on met en oeuvre un ou plusieurs diisocyanates monomères choisis parmi :

- le diisocyanato-1,3 propane,
- le diisocyanato-1,4 butane,
- le diisocyanato-1,5 pentane,
- le diisocyanato-1,6 hexane,
- le diisocyanato-1,4 éthyl-2 butane,
- le diisocyanato-1,5 méthyl-2 pentane,
- le diisocyanato-1,6 triméthyl-2,2,4 hexane,
- le diisocyanat-1,6 triméthyl-2,4,4 hexane,
- le diisocyanato-1,2 cyclohexane,
- le diisocyanato-1,4 cyclohexane,
- le bis(isocyanatométhyl)-1,2 cyclobutane,
- le bis (isocyanato-4 cyclohexyl)méthane,
- le triméthyl-3,3,5 isocyanatométhyl-5 isocyanato-1 cyclohexane,
- le bis(isocyanatométhyl)-1,4 benzène,

- le bis(isocyanatométhyl)-1,2 benzène.

6. Procédé selon l'une des revendications 1 à 5, caractérisé en ce que la réaction de biurétisation est effectuée dans un milieu solvant représentant de 20 à 80 % en poids du mélange réactionnel.

7. Procédé selon la revendication 6, caractérisé en ce que le/ou les solvants utilisés sont choisis parmi les alcoxyalcanes, les carboxylates d'alcoxyalcanes, les carboxylates de diol et les esters méthyliques et/ou éthyliques de l'acide phosphorique.

8. Procédé selon l'une des revendications 1 à 7, caractérisé en ce que la réaction de biurétisation est effectuée en l'absence de solvant ou en présence de faibles quantités de solvants représentant de 1 à 10 fois le poids de l'eau engagée.

9. Procédé selon l'une des revendications 1 à 8, caractérisé en ce que l'on opère sous une pression absolue supérieure ou égale à 1,2 bar, avec une pression partielle de gaz carbonique d'au moins 0,2 bar.

10. Procédé selon l'une des revendications 1 à 9 , caractérisé en ce que l'on opère à une température comprise entre 70° C et 200° C, et de préférence comprise entre 110° C et 150° C.

## Claims

1. Process for the preparation of an organic polyisocyanate containing a biuret group, which consists in reacting at least one aliphatic, cycloaliphatic or arylaliphatic diisocyanate and a biuret-forming agent, at a temperature of at least 70° C, characterised in that the reaction is carried out in the presence of an effective quantity of at least one carboxylic acid of general formula (I):

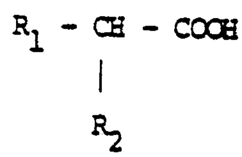

(I)

in which:
- $R_1$ represents:
  a hydrogen atom,
  a straight-chain or branched alkyl group containing 1 to 10 carbon atoms,
  a carboxyl group,
  a hydroxycarbonylalkyl group, the alkyl part of which, which may be straight-chain or branched, contains 1 to 9 carbon atoms, or
  an alkoxycarbonylalkyl group, the alkyl part of which, which may be straight-chain or branched, contains 1 to 9 carbon atoms, and
- $R_2$ represents:
  a hydrogen atom, or
  an alkyl group containing 1 to 4 carbon atoms,
- $R_1$ and $R_2$ may form, together and with the carbon atom to which they are attached, a cycloaliphatic ring containing 5 or 6 carbon atoms, which may be substituted with:
  one or two straight-chain or branched alkyl radical(s) containing 1 to 4 carbon atoms,
  one or two straight-chain or branched alkoxy radical(s) containing 1 to 4 carbon atoms,
  a carboxyl group,
  an alkoxycarbonyl group, the alkoxy part of which contains 1 to 4 carbon atoms,
  an acyl group containing 1 to 4 carbon atoms, or an acetoxy group;
  or of formula (II):

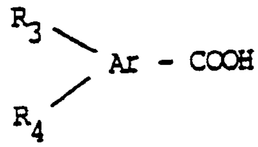

(II)

in which:

- Ar represents a benzene, pyridine or cyclopentanone ring,
- $R_3$ and $R_4$, which may be identical or different, represent:
  a hydrogen atom,
  a straight-chain or branched alkyl radical containing 1 to 4 carbon atoms,
  a straight-chain or branched alkoxy radical containing 1 to 4 carbon atoms,
  a halogen atom,
  an alkoxycarbonyl group, the alkoxy part of which contains 1 to 4 carbon atoms,
  an acyl group containing 1 to 4 carbon atoms, or an acetoxy group,
  and/or at least one anhydride of the acids of formula (I) or (II), and in that the quantity of carboxylic acid of formula (I) or (II) and/or of the anhydride of such an acid represents from 0.005% to 0.5%,preferably from 0.01% to 0.5%, of the weight of the diisocyanate monomer.

**2.** Process according to Claim 1, characterised in that the biuret-forming agent employed is water.

**3.** Process according to Claim 2, characterised in that the molar ratio of diisocyanate monomer:water is between 2 and 40 and preferably between 5 and 15.

**4.** Process according to one of Claims 1 to 3, characterised in that the carboxylic acid or the anhydride is chosen from acetic acid, propionic acid, isobutyric acid, benzoic acid and acetic anhydride.

**5.** Process according to one of Claims 1 to 4, characterised in that one or more diisocyanate monomers chosen from the following is (are) employed:

- 1,3-diisocyanatopropane,
- 1,4-diisocyanatobutane,
- 1,5-diisocyanatopentane,
- 1,6-diisocyanatohexane,
- 1,4-diisocyanato-2-ethylbutane,
- 1,5-diisocyanato-2-methylpentane,
- 1,6-diisocyanato-2,2,4-trimethylhexane,
- 1,6-diisocyanato-2,4,4-trimethylhexane,
- 1,2-diisocyanatocyclohexane,
- 1,4-diisocyanatocyclohexane,
- 1,2-bis(isocyanatomethyl)cyclobutane,
- bis(4-isocyanatocyclohexyl)methane,
- 3,3,5-trimethyl-5-isocyanatomethyl-1-isocyanatocyclohexane,
- 1,4-bis(isocyanatomethyl)benzene, and
- 1,2-bis(isocyanatomethyl)benzene.

**6.** Process according to one of Claims 1 to 5, characterised in that the biuret-forming reaction is carried out in a solvent medium representing from 20 to 80% by weight of the reaction mixture.

**7.** Process according to Claim 6, characterised in that the solvent(s) employed is (are) chosen from alkoxyalkanes, alkoxyalkane carboxylates, diol carboxylates and methyl and/or ethyl esters of phosphoric acid.

**8.** Process according to one of Claims 1 to 7, characterised in that the biuret-forming reaction is carried out in the absence of solvent or in the presence of small quantities of solvents representing from 1 to 10 times the weight of water employed.

**9.** Process according to one of Claims 1 to 8, characterised in that the reaction is carried out at an

absolute pressure greater than or equal to 1.2 bar, with a partial pressure of carbon dioxide of at least 0.2 bar.

**10.** Process according to one of Claims 1 to 9, characterised in that the reaction is carried out at a temperature of between 70° C and 200° C, and preferably between 110° C and 150° C.

## Patentansprüche

**1.** Verfahren zur Herstellung eines organischen Polyisocyanats mit Biuretgruppen, bestehend aus der Umsetzung wenigstens eines aliphatischen, cycloaliphatischen oder arylaliphatischen Diisocyanats und eines Biuretisierungsmittels bei einer Temperatur von wenigstens 70° C, dadurch gekennzeichnet, daß man in Gegenwart einer wirksamen Menge wenigstens einer Carbonsäure der allgemeinen Formel (I)

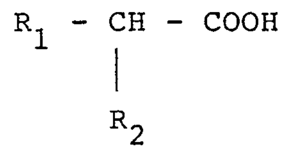

(I)

in der

- $R_1$
  . ein Wasserstoffatom,
  . eine 1 bis 10 Kohlenstoffatome enthaltende geradkettige oder verzweigte Alkylgruppe,
  . eine Carboxylgruppe,
  . eine Hydroxycarbonylalkylgruppe, deren geradkettiger oder verzweigter Alkylteil 1 bis 9 Kohlenstoffatome enthält, und
  . eine Alkoxycarbonylalkylgruppe, deren geradkettiger oder verzweigter Alkylteil 1 bis 9 Kohlenstoffatome enthält,
  bedeutet,
- $R_2$
  . ein Wasserstoffatom und
  . eine 1 bis 4 Kohlenstoffatome enthaltende Alkylgruppe
  bedeutet und
- $R_1$ und $R_2$ zusammen und mit dem Kohlenstoffatom, an das sie gebunden sind, einen 5 oder 6 Kohlenstoffatome enthaltenden cycloaliphatischen Ring bilden können, der durch
  . einen oder zwei 1 bis 4 Kohlenstoffatome enthaltende geradkettige oder verzweigte Alkylreste,
  . einen oder zwei 1 bis 4 Kohlenstoffatome enthaltende geradkettige oder verweigte Alkoxyreste,
  . eine Carboxylgruppe,
  . eine Alkoxycarbonylgruppe, deren Alkoxyteil 1 bis 4 Kohlenstoffatome enthält,
  . eine 1 bis 4 Kohlenstoffatome enthaltende Acylgruppe und
  . eine Acetoxygruppe
  substituiert sein kann,
  oder der Formel (II)

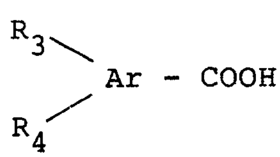

(II)

in der

- Ar einen Benzol-, Pyridin- oder Cyclopentanoncyclus bedeutet und
- $R_3$ und $R_4$ gleich oder verschieden sind und
  . ein Wasserstoffatom,
  . einen 1 bis 4 Kohlenstoffatome enthaltenden geradkettigen oder verzweigten Alkylrest,
  . einen 1 bis 4 Kohlenstoffatome enthaltenden geradkettigen oder verzweigten Alkoxyrest,

. ein Halogenatom,
. eine Alkoxycarbonylgruppe, deren Alkoxyteil 1 bis 4 Kohlenstoffatome enthält,
. eine 1 bis 4 Kohlenstoffatome enthaltende Acylgruppe und
. eine Acetoxygruppe

bedeuten,
und/oder wenigstens eines Anhydrids der Säuren der Formel (I) oder (II) arbeitet, und daß die Menge der Carbonsäure der Formel (I) oder (II) und/oder des Anhydrids einer solchen Säure 0,005 bis 0,5% und vorzugsweise 0,01 bis 0,5% des Gewichts des Diisocyanatmonomeren beträgt.

**2.** Verfahren nach Anspruch 1, dadurch gekennzeichnet, daß das verwendete Biuretisierungsmittel Wasser ist.

**3.** Verfahren nach Anspruch 2, dadurch gekennzeichnet, daß das Molverhältnis von Diisocyanatmonomerem/ Wasser zwischen 2 und 40 und vorzugsweise zwischen 5 und 15 beträgt.

**4.** Verfahren nach einem der Ansprüche 1 bis 3, dadurch gekennzeichnet, daß die Carbonsäure oder das Anhydrid aus Essig-, Propion-, Isobutter- und Benzoesäure und Essigsäureanhydrid ausgewählt ist.

**5.** Verfahren nach einem der Ansprüche 1 bis 4, dadurch gekennzeichnet, daß man ein oder mehrere Diisocyanatmonomere einsetzt, die aus

- 1,3-Propandiisocyanat,
- 1,4-Butandiisocyanat,
- 1,5-Pentandiisocyanat,
- 1,6-Hexandiisocyanat,
- 2-Ethyl-1,4-butandiisocyanat,
- 2-Methyl-1,5-pentandiisocyanat,
- 2,2,4-Trimethyl-1,6-hexandiisocyanat,
- 2,4,4-Trimethyl-1,6-hexandiisocyanat,
- 1,2-Cyclohexandiisocyanat,
- 1,4-Cyclohexandiisocyanat,
- 1,2-Bis(methylisocyanat)-cyclobutan,
- Bis(4-cyclohexylisocyanat)methan,
- 3,3,5-Trimethyl-5-isocyanatomethyl-1-isocyanatocyclohexan,
- 1,4-Bis(methylisocyanat)-benzol und
- 1,2-Bis(methylisocyanat)-benzol

ausgewählt sind.

**6.** Verfahren nach einem der Ansprüche 1 bis 5, dadurch gekennzeichnet, daß die Biuretisierungsreaktion in einem Lösungsmittelmedium durchgeführt wird, das 20 bis 80 Gew.% des Reaktionsgemischs ausmacht.

**7.** Verfahren nach Anspruch 6, dadurch gekennzeichnet, daß das oder die verwendeten Lösungsmittel aus Alkoxyalkanen, Alkoxyalkylcarboxylaten, Diolcarboxylaten und Methyl- und/oder Ethylestern der Phosphorsäure ausgewählt sind.

**8.** Verfahren nach einem der Ansprüche 1 bis 7, dadurch gekennzeichnet, daß die Biuretisierungsreaktion ohne Lösungsmittel oder in Gegenwart geringer Lösungsmittelmengen durchgeführt wird, die das 1- bis 10fache des Gewichts des eingesetzten Wassers betragen.

**9.** Verfahren nach einem der Ansprüche 1 bis 8, dadurch gekennzeichnet, daß man unter einem absoluten Druck von über oder gleich 1,2 bar mit einem Kohlendioxidpartialdruck von wenigstens 0,2 bar arbeitet.

**10.** Verfahren nach einem der Ansprüche 1 bis 9, dadurch gekennzeichnet, daß man bei einer Temperatur zwischen 70 und 200 °C und vorzugsweise zwischen 110 und 150 °C arbeitet.